(19) Europäisches Patentamt
European Patent Office
Office européen des brevets

(11) EP 0 941 370 B1

# (12) EUROPÄISCHE PATENTSCHRIFT

(45) Veröffentlichungstag und Bekanntmachung des Hinweises auf die Patenterteilung:
**12.06.2002 Patentblatt 2002/24**

(21) Anmeldenummer: **97950169.9**

(22) Anmeldetag: **11.11.1997**

(51) Int Cl.[7]: **C12Q 1/68**

(86) Internationale Anmeldenummer:
**PCT/EP97/06276**

(87) Internationale Veröffentlichungsnummer:
**WO 98/21362 (22.05.1998 Gazette 1998/20)**

(54) **STABILISIERTE WÄSSRIGE NUKLEOSIDTRIPHOSPHAT-LÖSUNGEN**

STABILIZED AQUEOUS NUCLEOSIDE TRIPHOSPHATE SOLUTION

SOLUTIONS AQUEUSES STABILISEES DE TRIPHOSPHATES DE NUCLEOSIDES

(84) Benannte Vertragsstaaten:
**AT BE CH DE DK ES FI FR GB IE IT LI NL SE**

(30) Priorität: **14.11.1996 DE 19647055**

(43) Veröffentlichungstag der Anmeldung:
**15.09.1999 Patentblatt 1999/37**

(73) Patentinhaber: **Roche Diagnostics GmbH**
**68298 Mannheim (DE)**

(72) Erfinder:
- **IHLENFELDT, Hans-Georg**
  **D-82393 Iffeldorf (DE)**
- **SCHMIDT, Axel**
  **D-80634 München (DE)**
- **MÜHLEGGER, Klaus**
  **D-82398 Polling (DE)**
- **LEITENBERGER, Volker**
  **D-82402 Seeshaupt (DE)**

(56) Entgegenhaltungen:
**EP-A- 0 632 134**
**EP-A- 0 649 909**
**EP-A- 0 751 226**
**WO-A-92/03556**
**WO-A-96/14405**
**FR-A- 2 674 253**
**US-A- 5 432 065**

- **AUSUBEL F M: "Current Protocols in Molecular Biology" 1987 , JOHN WILEY & SONS, INC. XP002062215 siehe Seite 3.4.1 - Seite 3.4.3**
- **SAMBROOK J ET AL.: "Molecular Cloning" 1989 , COLD SPRING HARBOUR LABORATORY PRESS , COLD SPRING HARBOUR XP002062216 siehe Seite 10.16 - Seite 10.17 siehe Seite 14.20 - Seite 14.21**
- **Sigma Katalog, 1996, S. 1559**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 941 370 B1

## Beschreibung

Die Erfindung betrifft stabile wäßrige Lösungen enthaltend Nukleosidtriphosphate, wobei die Lösung einen pH-Wert über 7.5 aufweist.

Nukleosidtriphosphate (NTP), wie Ribonukleosid-, Desoxy- und Didesoxynukleosidtriphosphate werden auf dem Gebiet der Biochemie und der Molekularbiologie vielfältig eingesetzt. Die Anwendungen betreffen zum großen Teil DNA- und RNA- aufbauende bzw. DNA- und RNA- vervielfältigende Reaktionen wie z.B. die Reverse Transkriptase-Polymerasekettenreaktion (RT-PCR), cycle Sequencing und die Nick-Translation. Bei der RT-PCR werden beispielsweise durch die Reverse Transkriptase in 5'-3'-Richtung DNA-Ketten synthetisiert, wobei als Matrix ein RNA-Strang dient. Bestimmte NTPs wie z.B. Didesoxy-Nukleosidtriphosphate (dd-NTP) können als Kettenterminatoren bei der Sequenzierung von DNA eingesetzt werden. Eine der wichtigsten Anwendungen für Desoxy-Nukleosidtriphosphate (d-NTP) ist der Einsatz in der Polymerase-Kettenreaktion (PCR). Die Stabilität der NTP-Lösungen ist dabei vor allem während der Lagerung unbedingt erforderlich. Die d-NTPs (d-ATP, d-CTP, d-GTP, d-TTP, d-UTP u.a.) werden üblicherweise als Na- oder Li-Salze und typischerweise in Konzentrationen von 0,1 mol/l aufbewahrt und in dieser Form kommerziell angeboten. Die pH-Werte liegen in der Regel bei physiologischem pH-Werten, d.h. zwischen ca. 7,0 bis 7,5.

Der Nachteil der derzeit zur Verfügung stehenden, d.h. käuflich zu erwerbenden NTP-Lösungen besteht insbesondere in der Instabilität der NTPs bei Lagerung bzw. thermischer Belastung. Die NTPs neigen dazu, sich im Laufe der Zeit zu den entsprechenden Diphosphaten bzw. Monophosphaten zu zersetzen. Insbesondere bei höheren Temperaturen nimmt der Triphosphatgehalt ab. Um ca. 2-3% nimmt der Triphosphatgehalt bereits bei einem pH-Wert von ca. 7.5 und bei einer Temperatur von 35°C innerhalb von 10 Tagen ab. Dahingegen ist bei Raumtemperatur nach 6 Wochen ledeglich eine Abnahme des Triphosphatgehaltes von ca. 1% zu beobachten. Der Zerfall der Triphosphate in wäßriger Lösung limitiert somit die Haltbarkeit der NTP-Lösungen. Daher gewähren z.B. die Anbieter von d-NTPs eine Haltbarkeit von d-NTP-Lösungen von lediglich zwölf Monaten. Es besteht jedoch der Bedarf an wässrigen Lösungen, die lediglich d-NTP und dies in hohen Konzentrationen aufweisen, die eine höhere Langzeitstabilität als die gegenwärtig zur Verfügung stehenden Lösungen aufweisen.

Versuche eine verbesserte Stabilität von Triphosphaten zu erreichen, bezogen sich bisher ausschließlich auf entsprechende Adenosintriphosphat-Lösungen. Die Stabilität des Adenosintriphosphates wurde dabei in Abhängigkeit vom pH-Wert untersucht. Dabei wurde gemäß dem Stand der Technik die Anwesenheit von Stabilisatoren als unbedingt erforderlich beschrieben. So wird die Stabilität von Adenosintriphosphat in wäßriger Lösung bei einem pH-Wert von vorzugsweise 8,3 bis 9,2 als optimal in Gegenwart von EDTA beschrieben (JP 64/003444/DERWENT 66-11664F). In Gegenwart der Stabilisatoren wie Guanidin / Aminoguanidin bzw. Creatinin wird ein pH-Wert von 9 bis 10 (JP 71/038270/DERWENT 71-722169bzw. JP 71/033592/DERWENT 71-631879), in Gegenwart von Methionin als Stabilisator ein pH-Wert von vorzugsweise 9 bis 10,5 (JP 67/019115/DERWENT 66-29019F), in Gegenwart der Stabilisatoren Phosphat und Sorbitol/Mannitol/Glycerol/Benzyl-Alkohol/PEG ein pH-Wert von 8 bis 11 (JP 67/015115/DERWENT 6-28392F) und in Gegenwart von Glycerol/$H_3PO_4$ ein pH-Wert von 3,7 (FR4078/DERWENT 66-22085F) beschrieben. Die Anwesenheit von Stabilisatoren in wäßrigen d-NTP-Lösungen kann jedoch für viele Anwendungen kritisch bzw. störend sein.

Für die radioaktive Random Primed Markierung ist ein sogenannter "Ready-to-go" Kit bekannt (Pharmacia). Bei diesem sind zwar alle Reaktionskomponenten vorgemischt und bereits für Einzel-Ansätze voraliquotiert und trocken stabilisiert (verglast). Der Nachteil dieser Form ist jedoch, daß keine Flexibilität bezüglich der Ansatzgröße gegeben ist, zusätzlich wird Zeit für die Auflösung der "verglasten" Komponenten benötigt, was den ganzen Prozeß verlangsamt und weniger reproduzierbar macht.

In EP 0 649 909 wird die Aufgabe gelöst, sämtlichen Reaktionskomponenten eines Kits zur Markierung für Nukleinsäuren bereits vermischt in flüssiger Form anzubieten. Allerdings wird die Lagerstabilität der Mischung nur erreicht, indem als Stabilisator Glycerin zugesetzt wird.

Die Aufgabe der vorliegenden Erfindung bestand daher darin, eine stabilisierte wässrige Lösung enthaltend NTP's ohne den Zusatz von irgend welchen Stabilisatoren zur Verftigung zu stellen. Die Aufgabe wurde erfindungsgemäß dadurch gelöst, daß wäßrige NTP-Lösungen einen pH-Wert von über ungefähr 7,5 aufweisen. Zu diesen Nukleotidtriphosphaten zählen Ribonukleosid-, Desoxy- und Didesoxynukleotidtriphosphate, wobei als Basen sowohl die fünf natürlich vorkommenden, als auch modifizierte Basen wie z.B. Isoguanine, Deazaverbindungen sowie deren Derivate in Frage kommen. Die Nukleosidtriphosphate können zudem mit Reportergruppen markiert sein. In der Regel weisen die erfindungsgemäßen Lösungen einen pH-Wert in einem Bereich von größer als 7,5 bis maximal 11 auf, Als besonders vorteilhaft erwies sich ein pH-Wert von ca. 8 bis 10. Die Einstellung des pH-Wertes kann sowohl teilhaft erwies sich ein pH-Wert von ca. 8 bis 10. Die Einstellung des pH-Wertes kann sowohl durch Basenzugabe (z.B. NaOH, KOH, LiOH) als auch durch Zugabe eines Puffers (z.B. Tris- Puffer, Na-Carbonat-Puffer, Phosphatpuffer) erfolgen.

Die Konzentration der NTP-Lösung beträgt vorteilhafterweise zwischen ca. 2 mmol/l und 200 mmol/l. Besonders bevorzugt ist eine Konzentration der NTPs von ca. 100 bis 150 mmol/l.

Ein besonders wichtiger Bestandteil dieser Erfindung sind stabile d-NTP-Lösungen. Die Stabilität dieser Lösungen erscheint vor allem im Hinblick auf die Anwendung in der Polymerasc-Kettenreaktion von Vorteil. Der pH-Wert der d-NTP-Lösung liegt in der Regel über ca. 7,5 und unter ca. pH 11. Als besonders vorteilhaft erwies sich ein pH-Wert zwischen ca. 8 bis 10. Die Konzentration der stabilen d-NTP-Lösung beträgt zwischen 2 mmol/l und 200 mmol/l. Besonders bevorzugt ist eine Konzentration der d-NTPs von 100 bis 150 mmol/l.

Überraschenderweise hat sich gezeigt, daß die Stabilität der NTPs ohne den Zusatz irgendwelcher Stabilisatoren bei pH-Werten von größer als 7,5 in wäßriger Lösung höher ist als in den bisher bekannten Lösungen, die einen pH-Wert von ca. 7,0 bis 7,5 aufweisen. Die Stabilität der d-NTPs in wäßriger Lösung erreicht ein Optimum bei einem pH-Wert von ca. 8 bis 10. Die Erhöhung des pH-Wertes ruft keine zusätzlichen Abbaureaktionen hervor, d.h. das Muster der Abbauprodukte bleibt bei den erfindungsgemäßen pH-Werten unverändert. Die Abbaureaktionen verlaufen sogar überraschenderweise bei höheren pH-Werten, wie beispielsweise 8,3 erheblich langsamer als bei physiologischen pH-Werten, wie beispielsweise 7,5.

Somit hat sich gezeigt, daß bei erhöhten pH-Werten keinerlei Nebenprodukte entstehen, die die Verwendung der d-NTPs z.B. für die PCR-Reakion beeinträchtigen könnten. Selbst nach ca. 90 Tagen bei einer Temperatur von 35°C ist der PCR-Funktionstest positiv. Der höhere pH-Wert ist für die PCR selbst unkritisch, da die meisten PCR-Amplifikationen sowieso bei pH-Werten größer als 8,0 durchgeführt werden. Somit erweisen sich beispielsweise wäßrige d-NTP -Lösungen, deren pH-Wert größer als ca. 7,5 und kleiner/gleich ca. 11 ist, zum einen als stabil und als vorteilhaft für die Verwendung für die PCR-Reaktion. Als besonders vorteilhaft erwies sich hier ein pH-Wert der d-NTP-Lösung zwischen 8 und 10.

Die erfindungsgemäßen stabilen NTP-Lösungen können für alle DNA- und RNA aufbauende bzw. DNA- und RNA vervielfältigende Reaktionen verwendet werden. Insbesondere kann die erfindungsgemäße stabile NTP-Lösung auch für die RT-PCR, für Nick Translation, Random Priming und für die Sequenzierung (cycle Sequencing) verwendet werden. Weiterhin erwiesen sich die erfindungsgemäßen stabilen NTP-Lösungen vorteilhaft im Hinblick auf eine längere Verwendungsdauer der NTPs. Das heißt, daß die erfindungsgemäßen stabilen Lösungen wesentlich länger gelagert werden können als die bisher verwendeten d-NTP-Lösungen.

Erläuterungen der Abbildungen

Abbildung 1: Abnahme des d-GTP-Gehaltes
Die Ahnahme der d-GTP-Konzentration bei einer Temperatur von 35°C wurde über einen Zeitraum von 140 Tagen bei pH-Werten von 7,5; 7,9 bzw. 8,4 verfolgt.

Abbildung 2: Abnahme des d-CTP-Gehaltes
Die Abnahme der d-CTP-Konzentration bei einer Temperatur von 35°C wurde über einen Zeitraum von 90 Tagen bei pH-Werten von 7,5; 7,9 bzw. 8,3 verfolgt.

Abbildung 3: Abnahme des d-TTP-Gehaltes
Die Abnahme der d-TTP-Konzentration bei einer Temperatur von 35°C wurde über einen Zeitraum von 90 Tagen bei pH-Werten von 7,5; 7,9 bzw. 8,3 verfolgt.

Abbildung 4: Abnahme des d-UTP-Gehaltes
Die Abnahme der d-UTP-Konzentration bei einer Temperatur von 35°C wurde über einen Zeitraum von 90 Tagen bei pH-Werten von 7,5; 7,9 bzw. 8,3 verfolgt.

Abbildung 5: Abnahme des d-ATP-Gehaltes
Die Abnahme der d-ATP-Konzentration bei einer Temperatur von 35°C wurde über einen Zeitraum von 65 Tagen bei pH-Werten von 7,5; 7,9 bzw. 8,4 verfolgt.

Abbildung 6: Triphosphatgehalt in Abhängigkeit vom pH-Wert

Abbildung 7: pH-Abhängigkeit der Stabilität von UTP

Abbildung 8: pH-Abhängigkeit der Stabilität von UDP

Abbildung 9: pH-Abhängigkeit der Stabilität von ATP

Abbildung 10: pH-Abhängigkeit der Stabilität von ADP

Abbildung 11: pH-Abhängigkeit der Stabilität von 7-deaza-deoxy-GTP

Abbildung 12: pH-Abhängigkeit der Stabilität von 7-deaza-deoxy-GTP

Abbildung 13: die Abhängigkeit der Stabilität von dATP von der Konzentration der Lösung bei pH=8.3 wobei c=100 mmol/l, 10 mmol/l und 2 mmol/l

Abbildung 14: die Abhängigkeit der Stabilität von dATP von der Konzentration der Lösung bei pH=8,3 wobei c=100 mmol/l, 10 mmol/l und 2 mmol/l.

Abbildung 15: die Abhängigkeit der Stabilität von dCTP von der Konzentration der Lösung bei pH=8.3 wobei c=100 mmol/l, 10 mmol/l und 2 mmol/l.

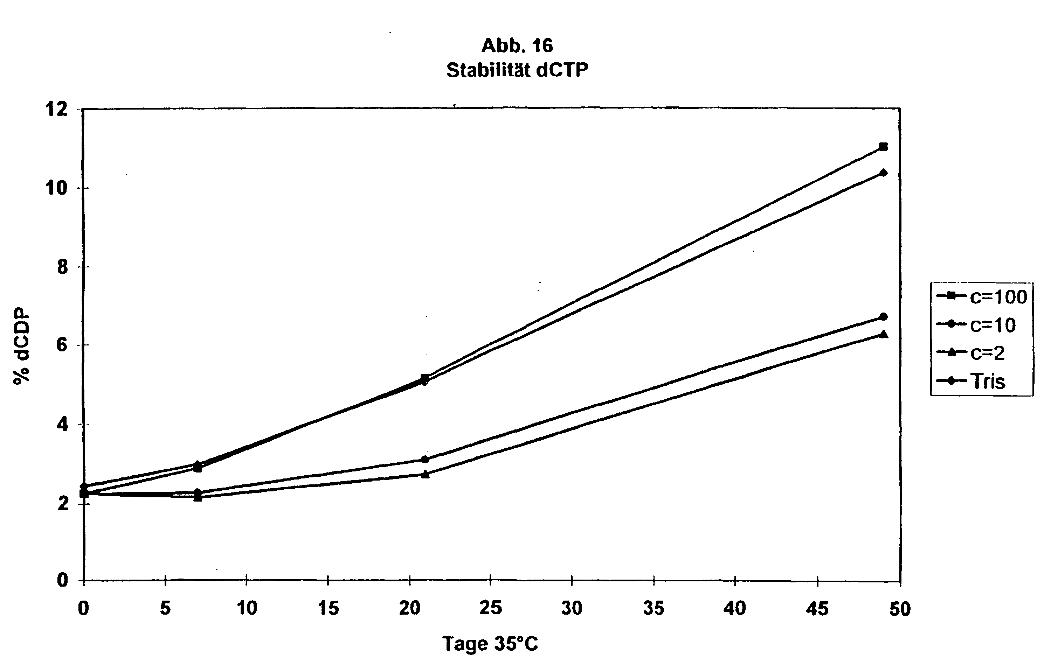

Abbildung 16: die Abhängigkeit der Stabilität von dCTP von der Konzentration der Lösung bei pH=8.3 wobei c=100 mmol/l, 10 mmol/l und 2 mmol/l.

Die folgenden Beispiele erläutern die Erfindung weiter:

Beispiel 1:

Herstellung einer erfindungsgemäßen stabilen d-NTP-Lösung

d-NTPs wurden über Anionenchromatographie mit Hilfe eines Salzgradienten aufgereinigt und durch Umkehrosmose entsalzt. Zur Entfernung von DNAsen/RNAsen schließt sich eine Ultrafiltration an (Ausschlußgrenze 1000 - 5000 D). Die Konzentration der Lösung wird dann mit Steril-Wasser typischerweise auf 100 mM eingestellt. Der pH-Wert wird durch Zugabe von Basen (Alkali/Erdalkali/Ammonium-Hydroxid; Amine), wie in der Regel NaOH auf den entsprechenden pH-Wert (>7,5) eingestellt.

Beispiel 2:

Abbau des Triphosphaies bei verschiedenen pH-Werten

d-NTP-Lösungen der Konzentration 100 bis 110 mmol/l wurden mit Natronlauge auf pH-Werte zwischen 7,5 und 8,3 eingestellt. Die Probe wurde bei 35°C, 22°C, 4°C und -20°C gelagert. Zu verschiedenen Zeitpunkten wurden Aliquots entnommen und die Reinheit mittels HPLC untersucht. Der Anteil des Tri-, Di,- und Monophosphatgehaltes sowie der freien Base wurde durch Integration der Flächen bestimmt.

Die Abnahme des Triphosphatgehaltes ist pH-abhängig. Bei allen untersuchten Nukleotiden ist die Abnahme bei ca. pH 8,3 am langsamsten. (Abb. 1-5)

D.h. selbst bei höheren pH-Werten wie 8,3 sind keine zusätzlichen Peaks, die aufZersetzungsproduktc schließen ließen, im HPLC-Chromatogramm zu sehen.

Beispiel 3:

Bestimmung des pH-Optimums der d-NTPS

d-NTP-Lösungen (dCTP, dTTP, dUTP) der Konzentration 100 bis 110 mmol/l wurden mit Natronlauge auf pH-Werte zwischen 7,5 und 12 (d-ATP, dGTP nicht pH 7,9 und 8,3) eingestellt. Die Probe wurde bei 35°C 35 Tage belastet und anschließend die Reinheit mittels HPLC untersucht. Der Anteil des Tri-, Di,- und Monophosphatgehaltes sowie der freien Base wurde durch Integration der Flächen bestimmt.

Bei allen untersuchten d-NTPs liegt das Optimum in einem Bereich zwischen pH 9,0 und 11,0. Bis pH 12 tritt nur geringfügiger Abbau ein (Ausnahme d-CTP, das bei pH 12 desaminiert wird. wodurch d-UTP entsteht) (siehe Abb. 6, 7, 8, 9, 10, 11, 12).

Beispiel 4:

Berechnung der Stabilisierung von d-NTPs bei pH 8,3 im Vergleich mit pH 7,5

Aus drei unabhängigen Belastungsversuchen von d-NTPs bei pH 8.3 und 7,5 bei 35°C mit Probennahmen in Zeiträumen zwischen 7 und 89 Tagen wurde die Stabilisierung nach folgender Formel abgeschätzt:

$$\frac{\Delta Gehalt(pH8,3) - \Delta Gehalt(7,5)}{\Delta Gehalt(pH7,5)} \; x\ 100$$

Mit

$$\Delta Gehalt(pH) = Gehalt(t{=}0) - Gehalt(t)$$

Für die einzelnen Nukleotide ergaben sich folgende Stabilisierungen in Prozent bei einem pH-Wert von 7,5 im Vergleich zu einem pH-Wert von 8,3 (Tabelle 1):

Tabelle 1:

| Nucleotid | Mittelwert | Maximalwert | Minimalwert |
|---|---|---|---|
| d-ATP | 19% | 33% | 9% |
| d-CTP | 20 % | 37% | 10% |
| d-GTP | 21% | 30% | 12% |
| d-TTP | 5% | 1.7% | 26% |
| d-UTP | 5% | 15% | 25% |

Beispiel 5:

Stabilisierung bei Raumtemperatur

Nach 204 Tagen (20°C) zeigt sich der Unterschied der pH-Stabilisierung (im Echtzeitmodell). Bei d-ATP-Lösungen nimmt der Triphosphatgehat beispielsweise bei pH 7,5 um ca. 7,6 %, bei pH 8,3 um ca. 6.3% ab (Differenz 17 %). Bei d-GTP-Lösungen nimmt der Triphosphatgehalt beispielsweise bei pH 7,5 um ca. 6,8%, bei pH 8,3 um ca. 5,2 % ab (Differenz 23%)

**Patentansprüche**

1. Stabile wäßrige Lösung enthaltend Nukleosidtriphosphate, wobei der pH-Wert der Lösung über 7,5 liegt, wobei die Lösung frei von weiteren stabilisierenden Agenzien ist und wobei nach ca. 90 Tagen bei einer Temperatur von 35°C der PCR-Funktionstest positiv ist.

2. Stabile wäßrige Lösung gemäß Anspruch 1, **dadurch gekennzeichnet, daß** es sich bei den Nukleosidtriphosphaten um modifizierte Nukleosidtriphosphate handelt.

3. Stabile wäßrige Lösung gemäß Anspruch 1 oder 2, wobei der pH-Wert in einem Bereich zwischen 7,5 und 11 liegt.

4. Stabile wäßrige Lösung gemäß Anspruch 1,2 oder 3, wobei die Konzentrationen der Nukleosidtriphosphate ca. 2 bis 200 mmol/l beträgt.

5. Stabile wäßrige Lösung gemäß einem der Ansprüche 1 bis 4, wobei die Lösung Desoxy-Nukleosidtriphosphate enthält.

6. Stabile wäßrige Lösung gemäß der Ansprüche 1 bis 5 enthaltend eine bei bzw. über pH 7,5 puffernde Substanz.

7. Verwendung einer stabilen wäßrigen Lösung gemäß der Ansprüche 1 bis 6 zu einer DNA- und/oder RNA- aufbauenden Reaktion.

8. Verwendung einer stabilen wäßrigen Lösung gemäß der Ansprüche 1 bis 6 zur Vervielfältigung von DNA- und/oder RNA-Sequenzen bzw. -Fragmenten.

9. Verwendung einer stabilen wäßrigen Lösung gemäß der Ansprüche 1 bis 6 zur spezifischen Vervielfältigung von Nukleinsäurefragmenten in Gegenwart eines Enzyms mit reverser Transkriptase-Aktivität.

**10.** Verwendung einer stabilen wäßrigen Lösung gemäß der Ansprüche 1 bis 6 zum cycle Sequencing von Nukleinsäuren.

**11.** Verwendung einer stabilen wäßrigen Lösung gemäß der Ansprüche 1 bis 6 zur spezifischen Vervielfältigung von Desoxynukleinsäuresequenzen bzw. -fragmenten.

**12.** Verwendung einer stabilen wäßrigen Lösung gemäß der Ansprüche 1 bis 6 zum Random Priming.

**13.** Verwendung einer stabilen wäßrigen Lösung, gemäß der Ansprüche 1 bis 6 zur Nick Translation.

## Claims

**1.** Stable aqueous solution containing nucleoside triphosphates, wherein the pH value of the solution is above 7.5, the solution is free of additional stabilizing agents and the PCR function test is positive after ca. 90 days at a temperature of 35°C.

**2.** Stable aqueous solution as claimed in claim 1, wherein the nucleoside triphosphates are modified nucleoside triphosphates.

**3.** Stable aqueous solution as claimed in claim 1 or 2, wherein the pH value is in a range between 7.5 and 11.

**4.** Stable aqueous solution as claimed in claim 1, 2 or 3, wherein the concentration of the nucleoside triphosphates is ca. 2 to 200 mmol/l.

**5.** Stable aqueous solution as claimed in one of the claims 1 to 4, wherein the solution contains deoxynucleoside triphosphates.

**6.** Stable aqueous solution as claimed in claims 1 to 5 containing a substance which buffers at or above pH 7.5.

**7.** Use of a stable aqueous solution as claimed in claims 1 to 6 for a DNA and/or RNA synthesizing reaction.

**8.** Use of a stable aqueous solution as claimed in claims 1 to 6 to replicate DNA and/or RNA sequences or fragments.

**9.** Use of a stable aqueous solution as claimed in claims 1 to 6 to specifically replicate nucleic acid fragments in the presence of an enzyme with reverse transcriptase activity.

**10.** Use of a stable aqueous solution as claimed in claims 1 to 6 for the cycle sequencing of nucleic acids.

**11.** Use of a stable aqueous solution as claimed in claims 1 to 6 for the specific replication of deoxynucleic acid sequences or fragments.

**12.** Use of a stable aqueous solution as claimed in claims 1 to 6 for random priming.

**13.** Use of a stable aqueous solution as claimed in claims 1 to 6 for nick translation.

## Revendications

**1.** Solution aqueuse stable contenant des triphosphates de nucléoside, dans laquelle la valeur du pH de la solution étant supérieure à 7,5, dans laquelle la solution est exempte d'agents stabilisants supplémentaires et dans laquelle le test de fonction PCR est positif après environ 90 jours à une température de 35°C.

**2.** Solution aqueuse stable selon la revendication 1, **caractérisée en ce que**, quant aux triphosphates de nucléoside, il s'agit de triphosphates de nucléoside modifiés.

3. Solution aqueuse stable selon la revendication 1 ou 2, dans laquelle la valeur du pH est dans une plage comprise entre 7,5 et 11.

4. Solution aqueuse stable selon la revendication 1, 2 ou 3, dans laquelle les concentrations en triphosphates de nucléoside sont d'environ 2 à 200 mmoles/l.

5. Solution aqueuse stable selon l'une quelconque des revendications 1 à 4 dans laquelle la solution contient des triphosphates de désoxynucléoside.

6. Solution aqueuse stable selon les revendications 1 à 5, contenant une substance tampon à pH de 7,5 ou plus.

7. Utilisation d'une solution aqueuse stable selon les revendications 1 à 6, pour une réaction de synthèse d'ADN et/ou d'ARN.

8. Utilisation d'une solution aqueuse stable selon les revendications 1 à 6, pour une réplication de séquences ou fragments d'ADN et/ou d'ARN.

9. Utilisation d'une solution aqueuse stable selon les revendications 1 à 6, pour une réplication spécifique de fragments d'acide nucléique en présence d'une enzyme ayant une activité de transcriptase inverse.

10. Utilisation d'une solution aqueuse stable selon les revendications 1 à 6, pour le séquençage cyclique d'acides nucléiques.

11. Utilisation d'une solution aqueuse stable selon les revendications 1 à 6, pour une réplication spécifique de séquences ou fragments d'acide désoxynucléique.

12. Utilisation d'une solution aqueuse stable selon les revendications 1 à 6, pour l'amorçage aléatoire ("random priming").

13. Utilisation d'une solution aqueuse stable selon les revendications 1 à 6, pour une traduction de coupure ("nick translation").

Abb. 1
Abnahme d-GTP

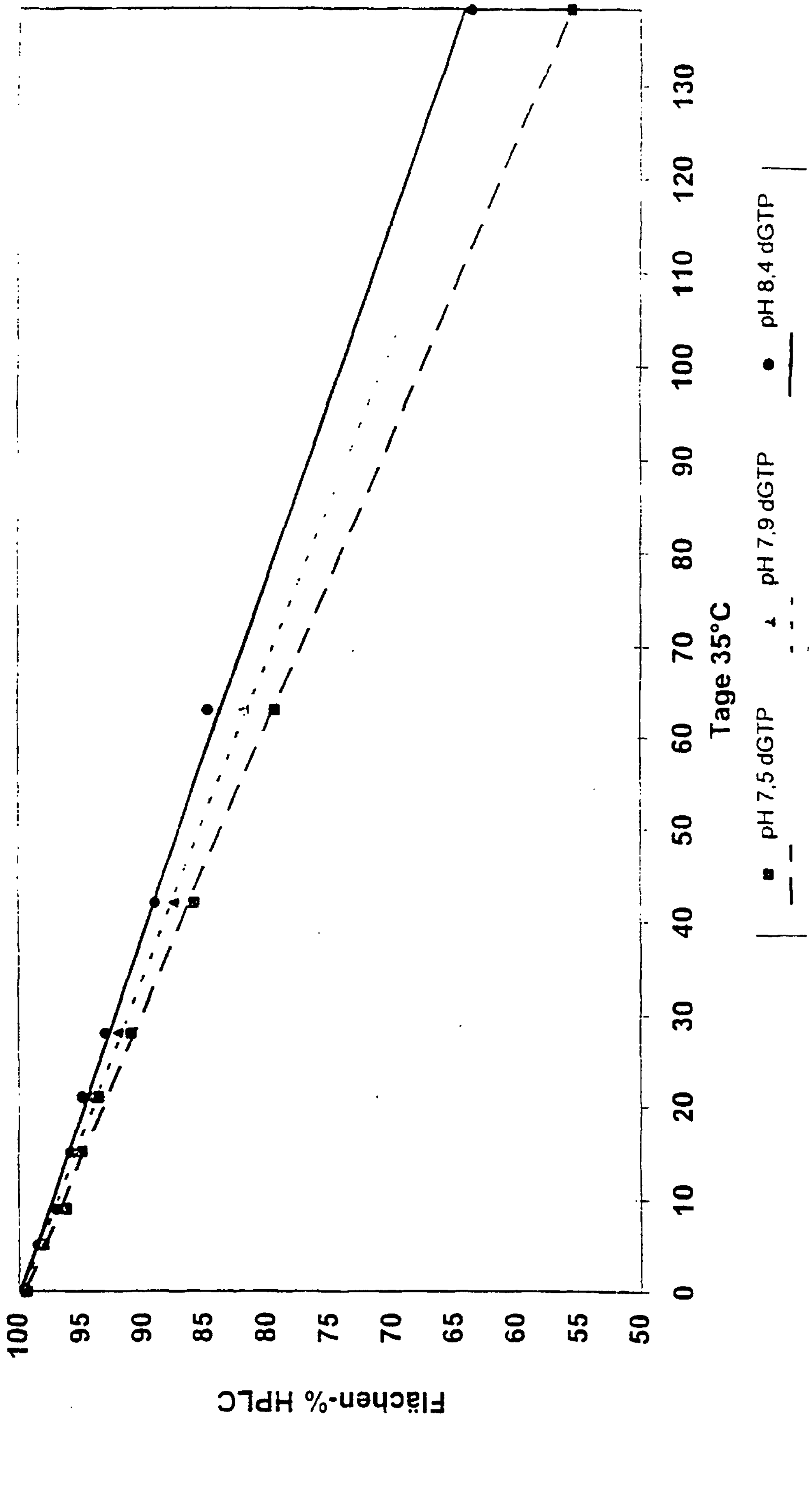

Abb. 2
Abnahme d-CTP

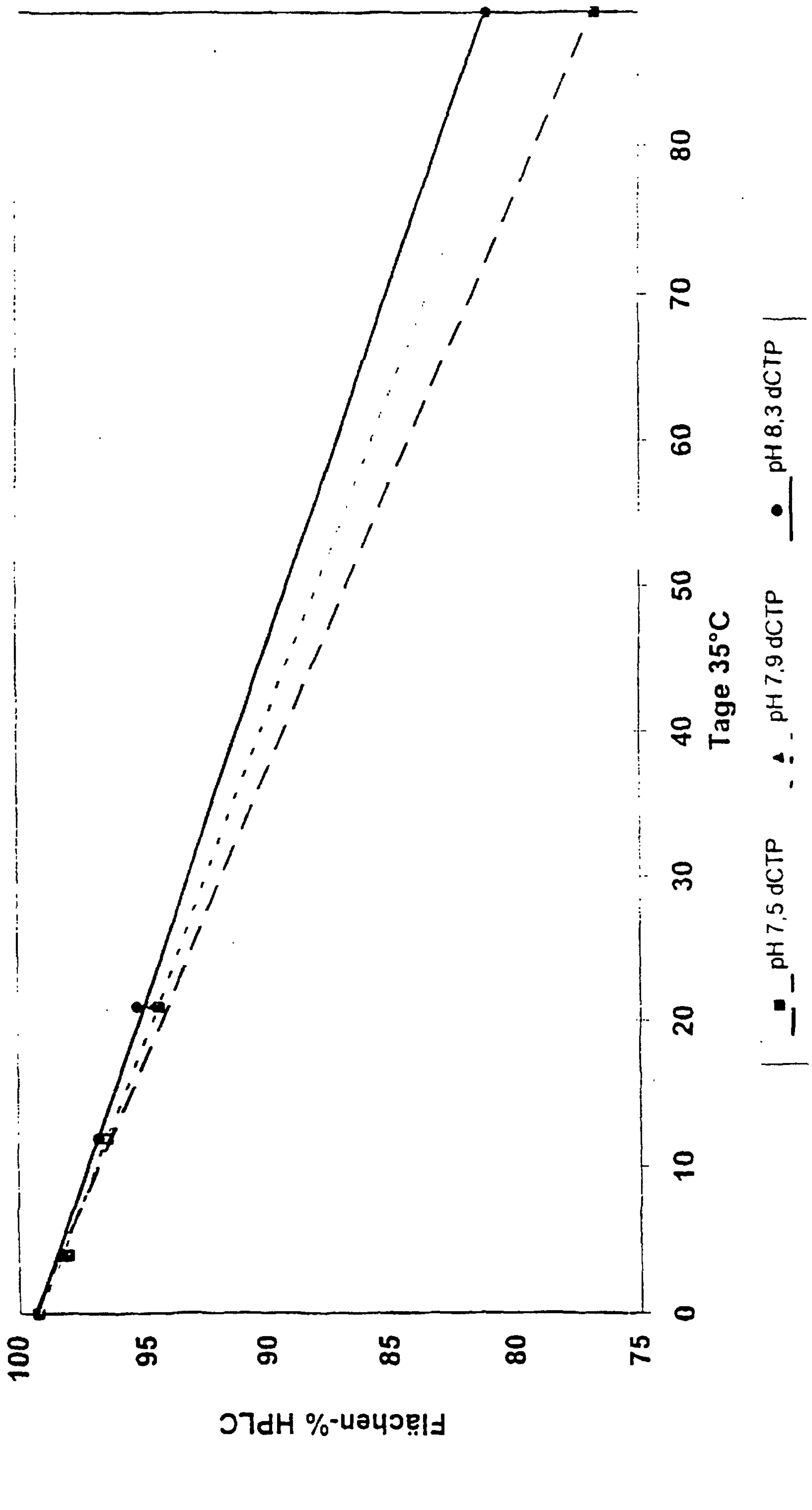

Abb. 3
Abnahme d-TTP

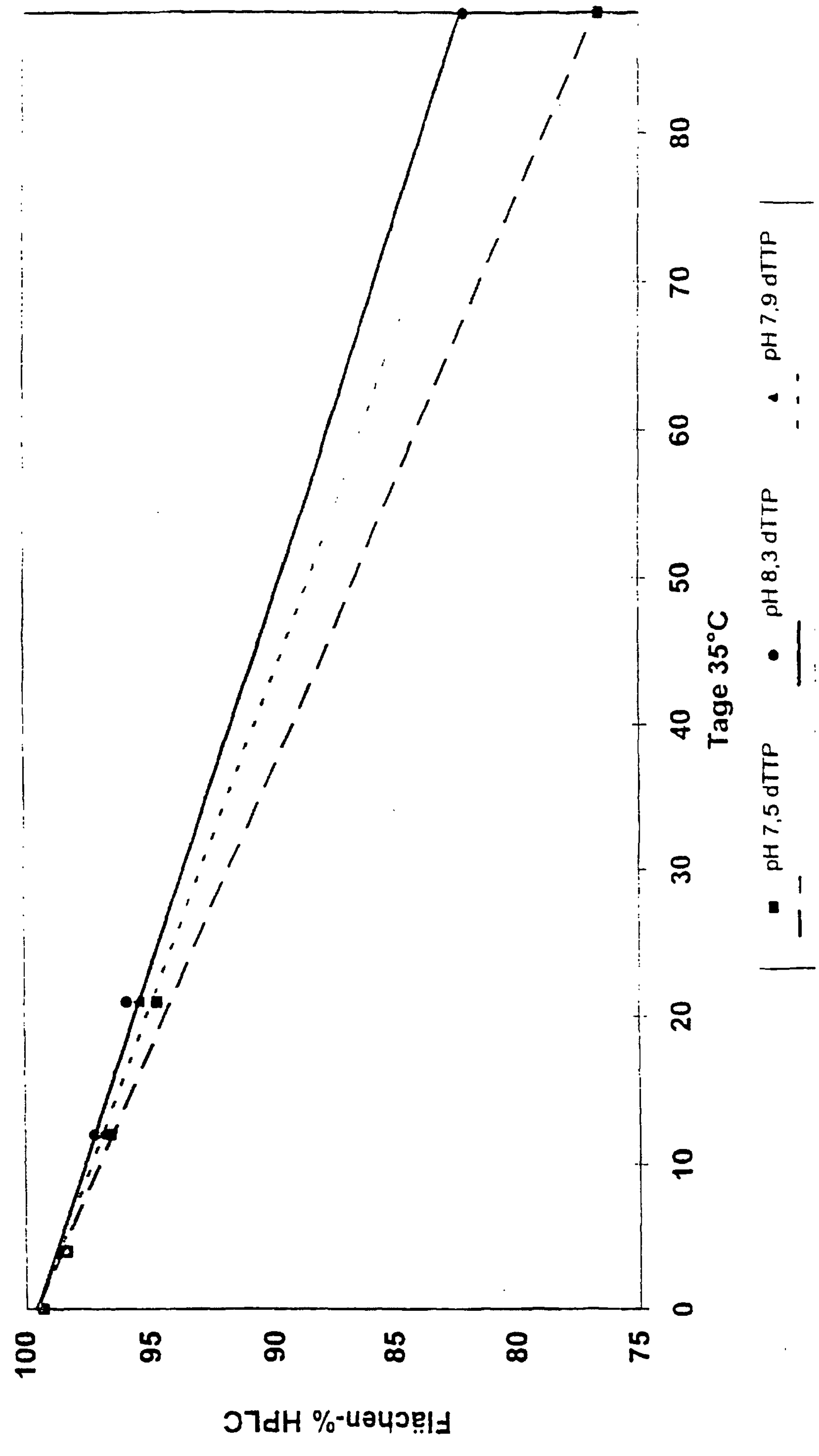

Abb. 4
Abnahme d-UTP

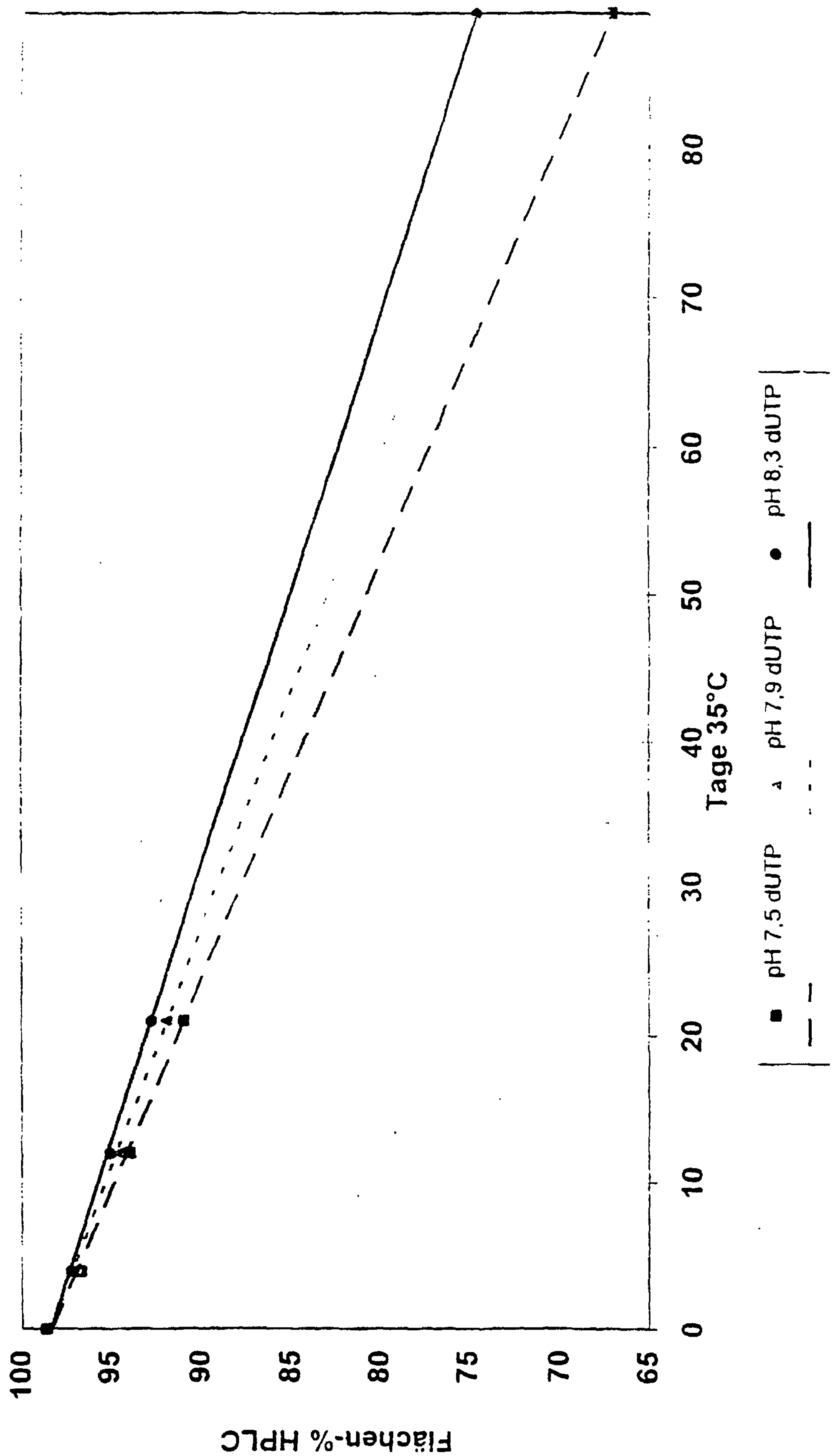

**Abb. 5**

**Abnahme d-ATP**

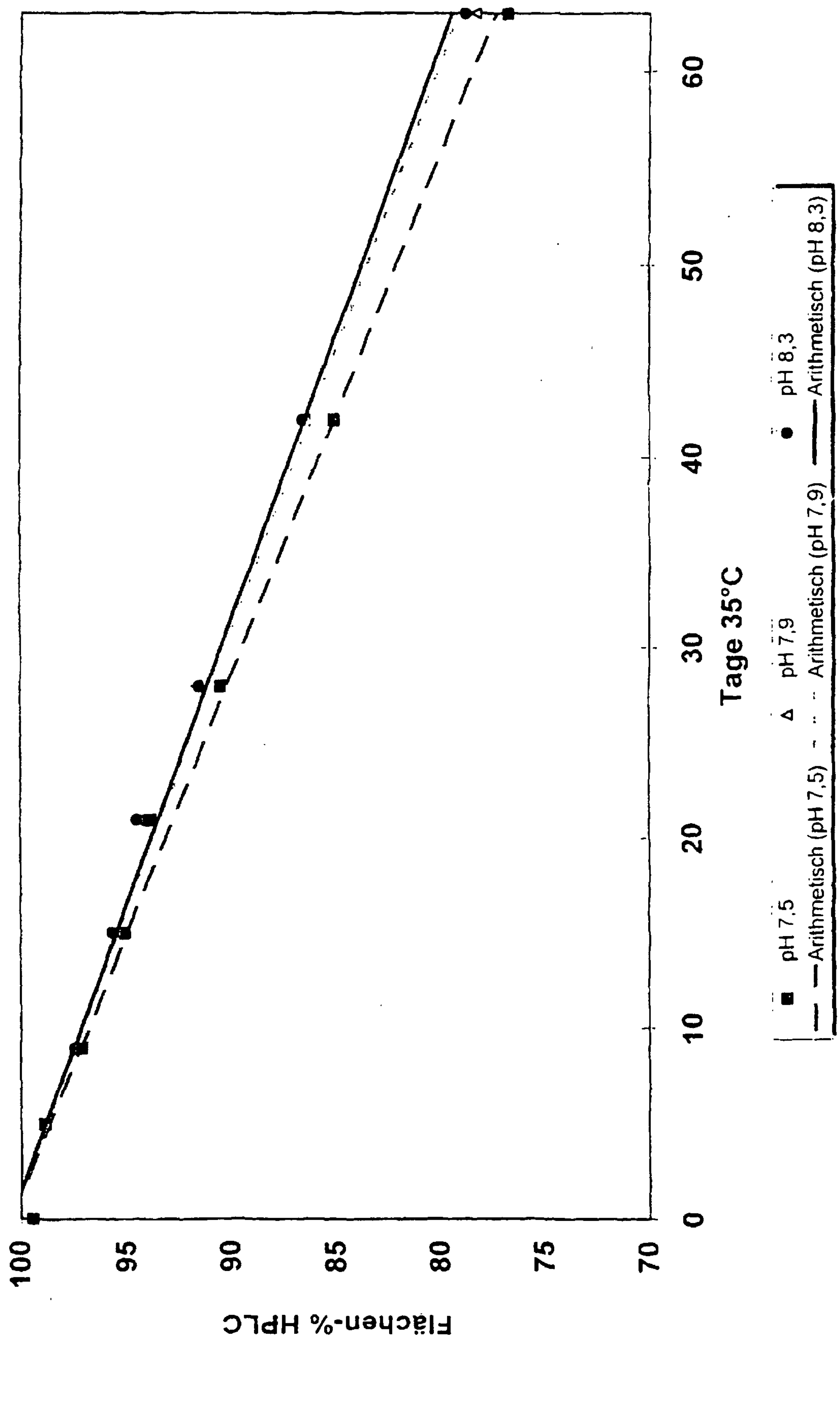

Abb. 6

pH-Optimum von d-NTPs
(Triphosphatgehalt nach 35 Tagen 35°C)

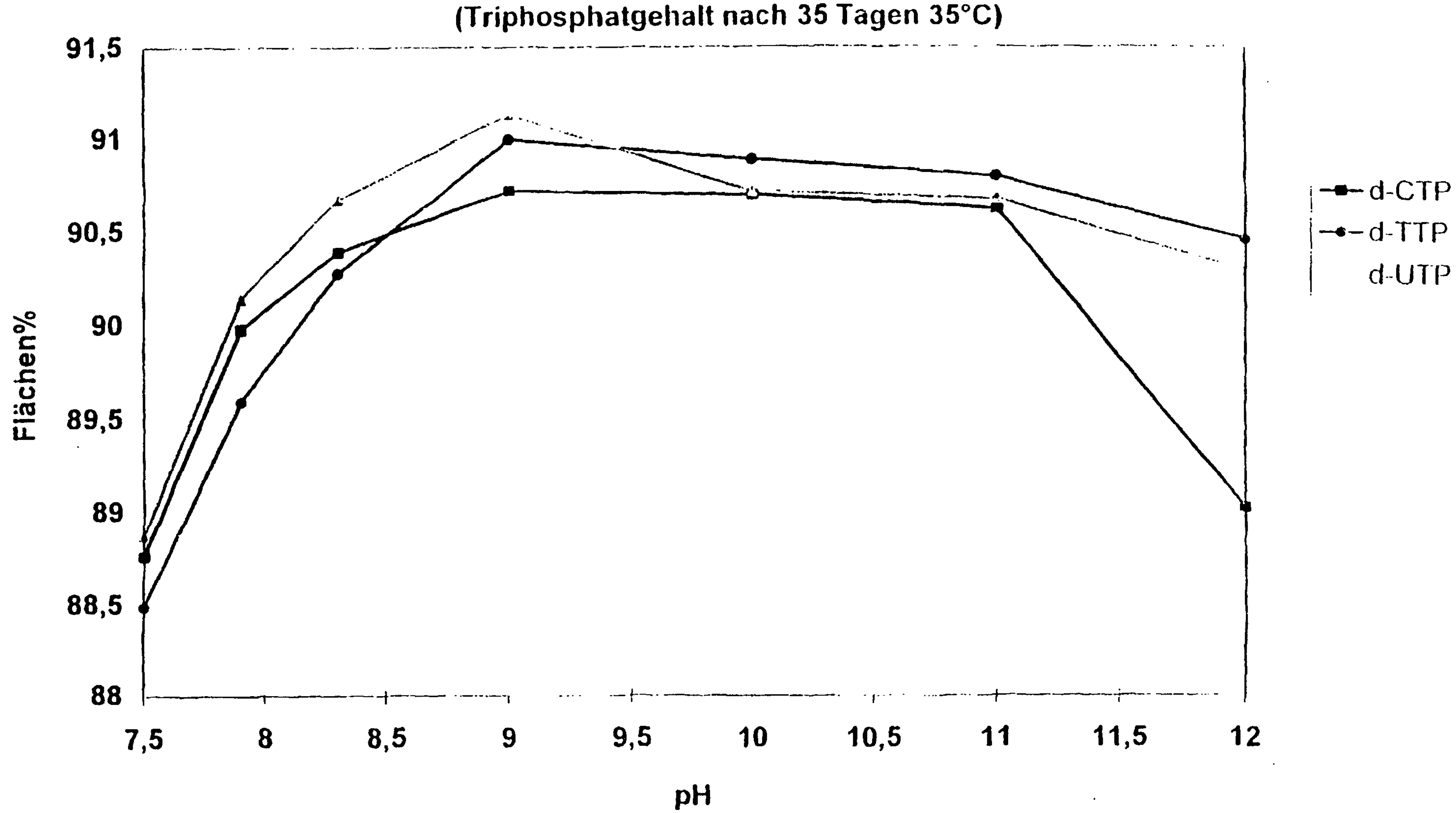

Abb. 7
pH-Stabilität UTP

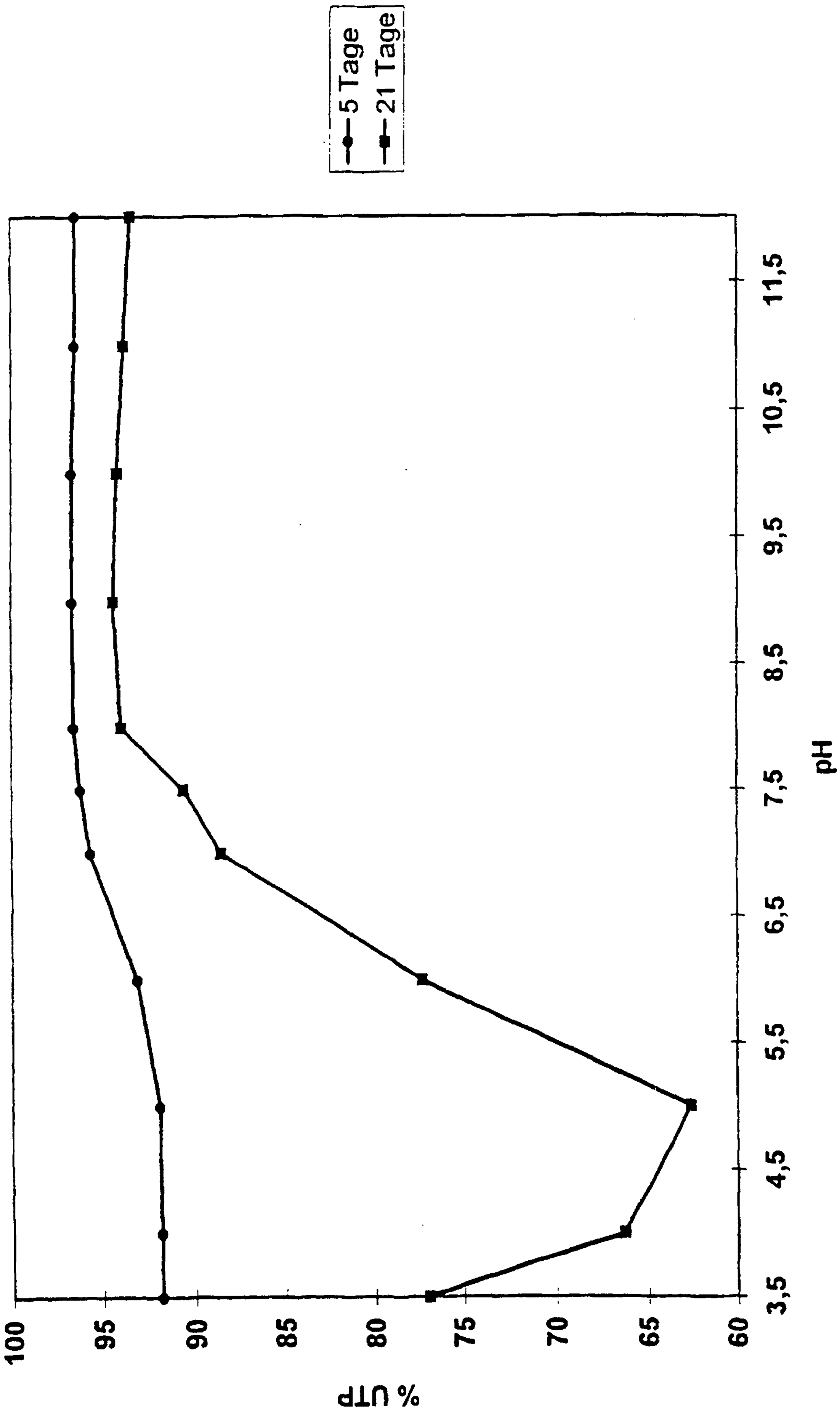

Abb. 8
Stabilität UDP

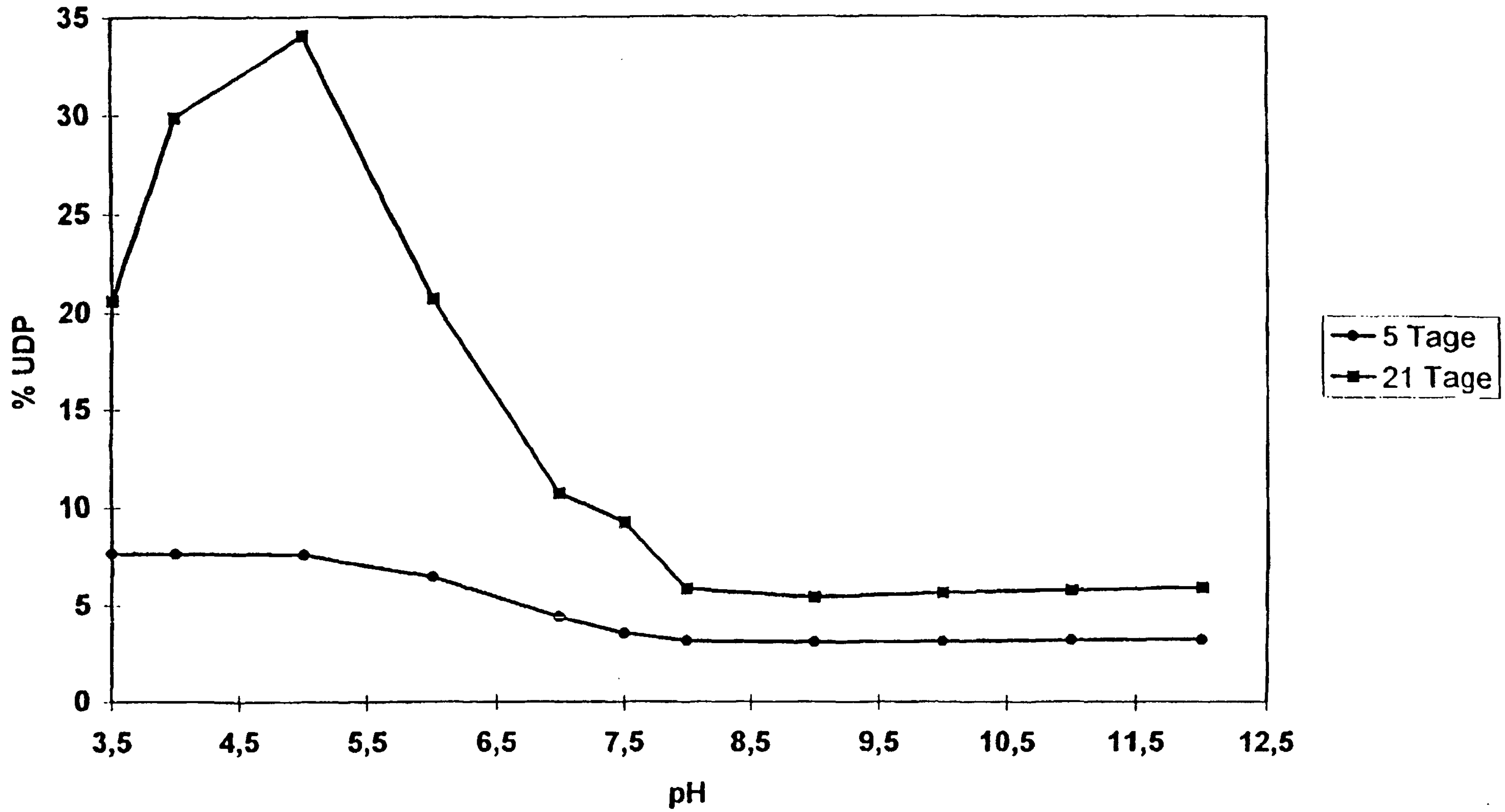

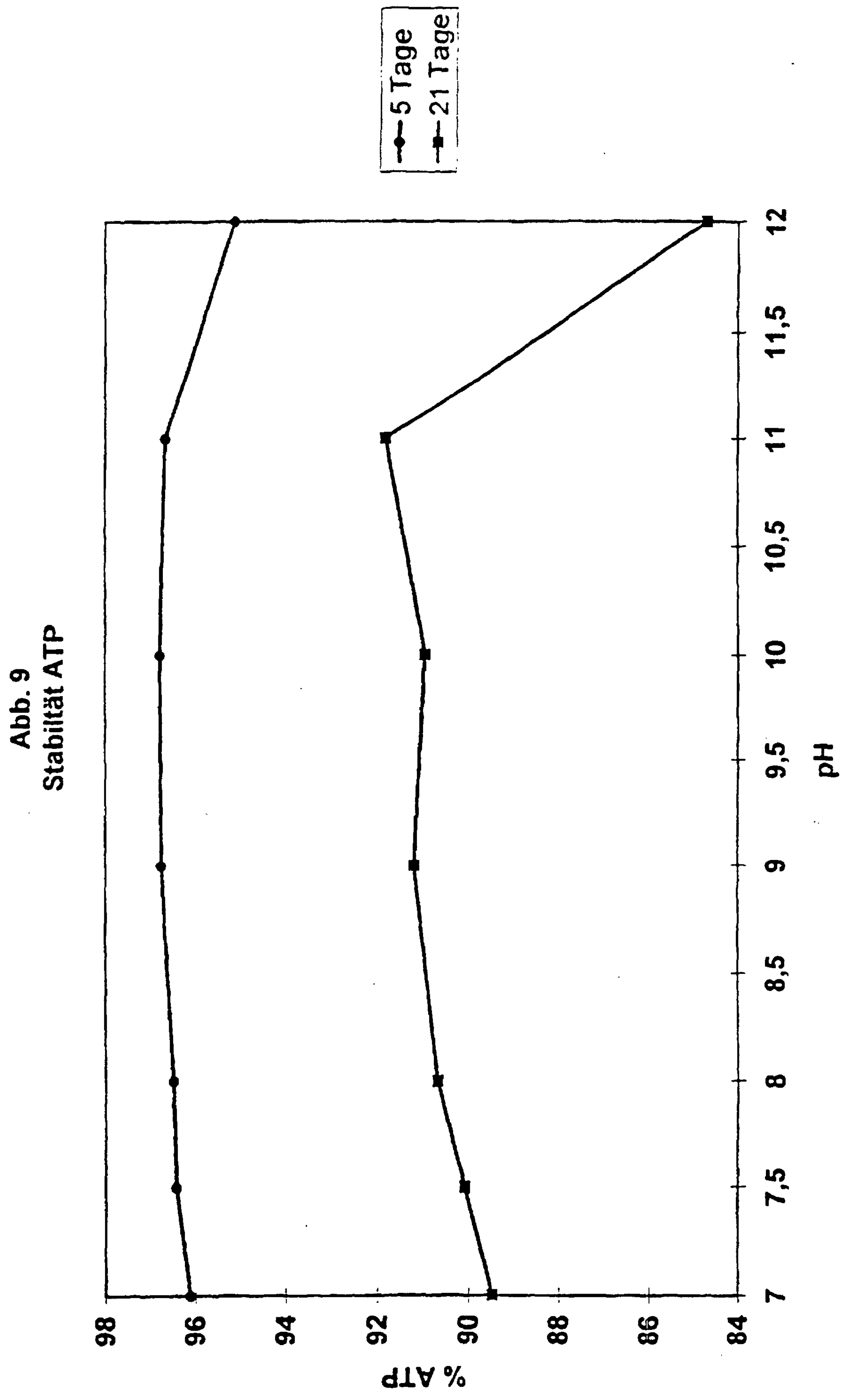
Abb. 9
Stabilität ATP
5 Tage
21 Tage
% ATP
98
96
94
92
90
88
86
84
7
7,5
8
8,5
9
9,5
10
10,5
11
11,5
12
pH

Abb. 10
Stabilität ADP

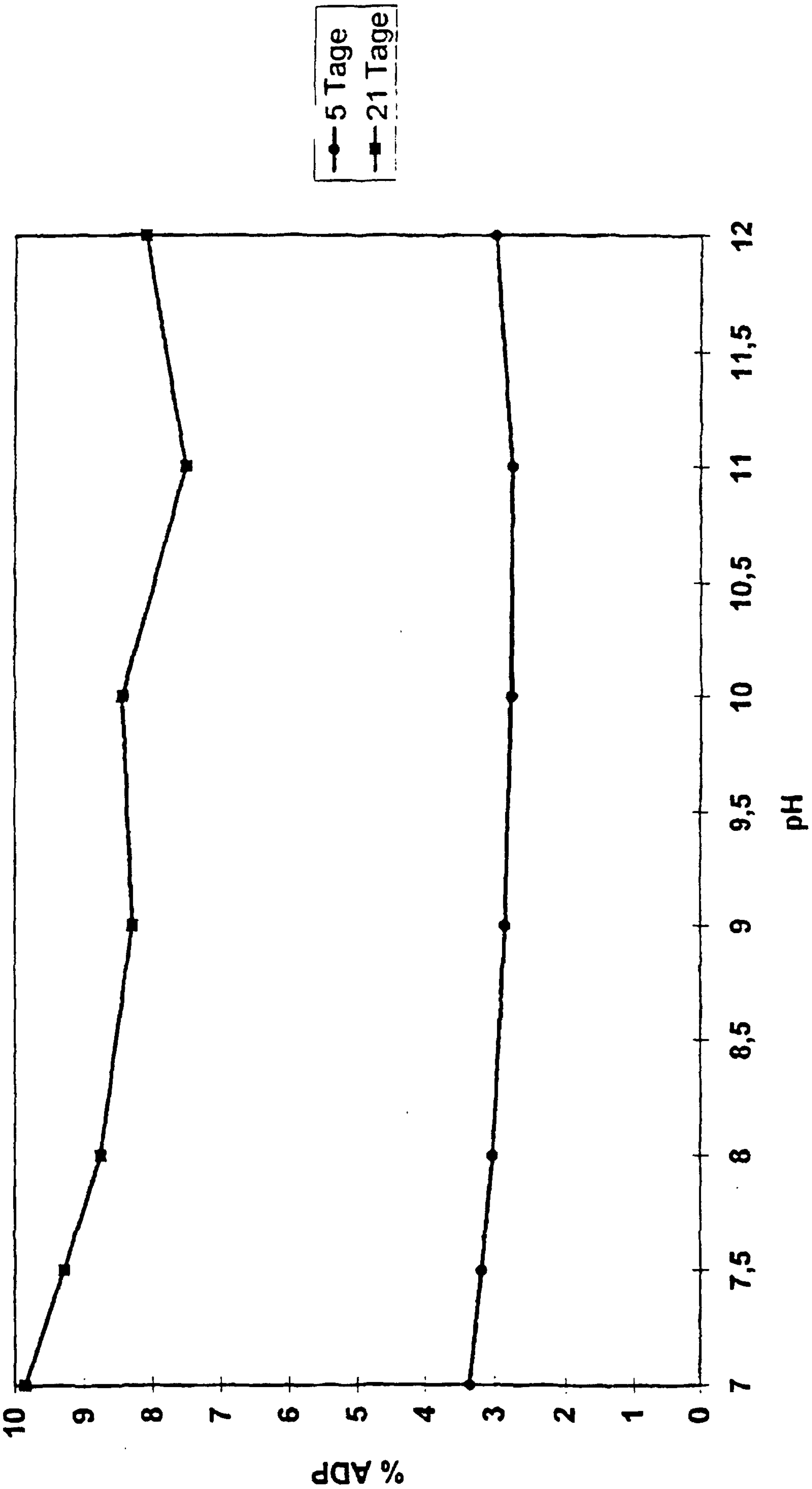

Abb. 11
Stabilität 7-deaza-d-GTP

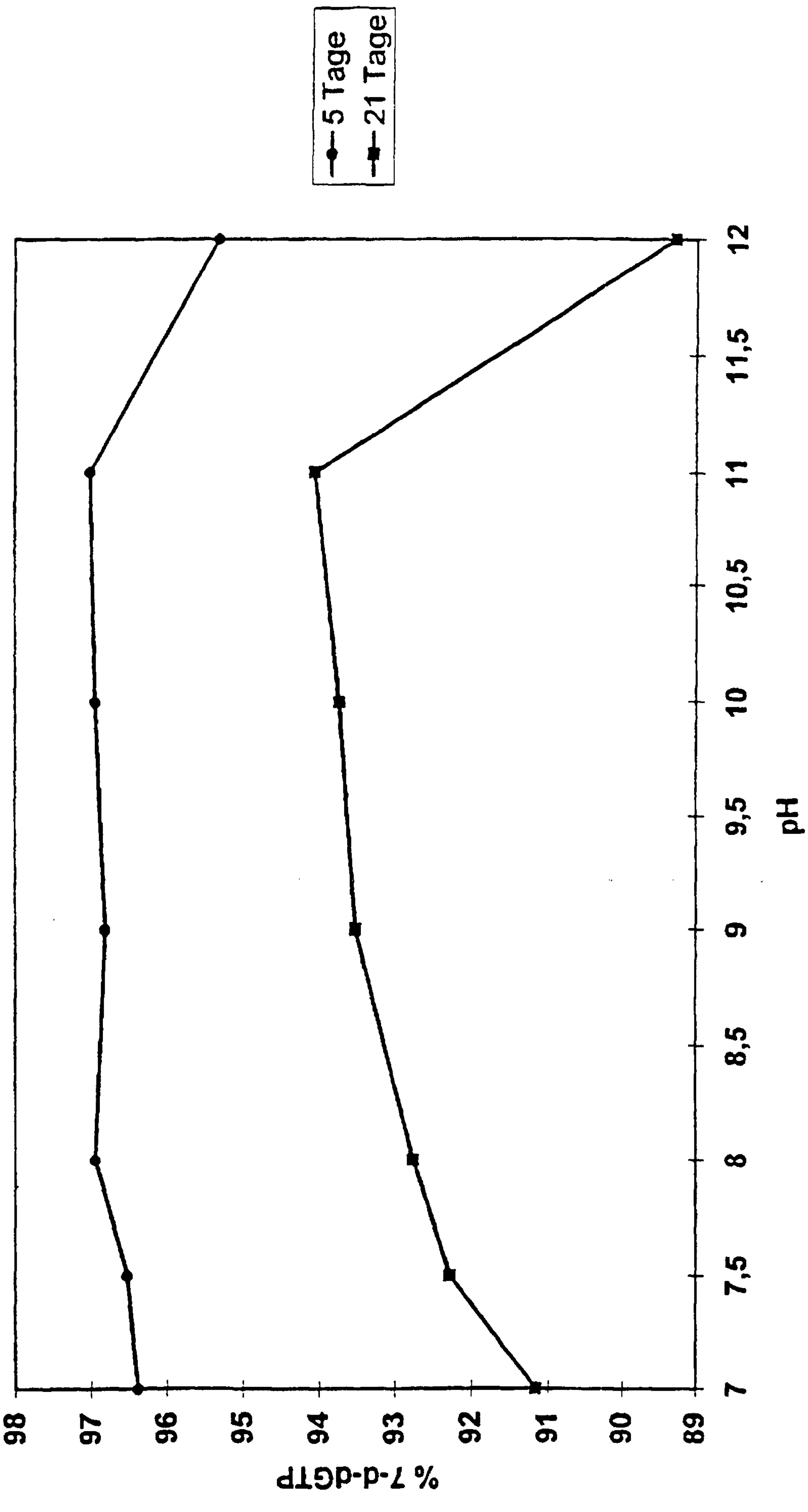

Abb. 12
Stabilität 7-deaza-d-GTP

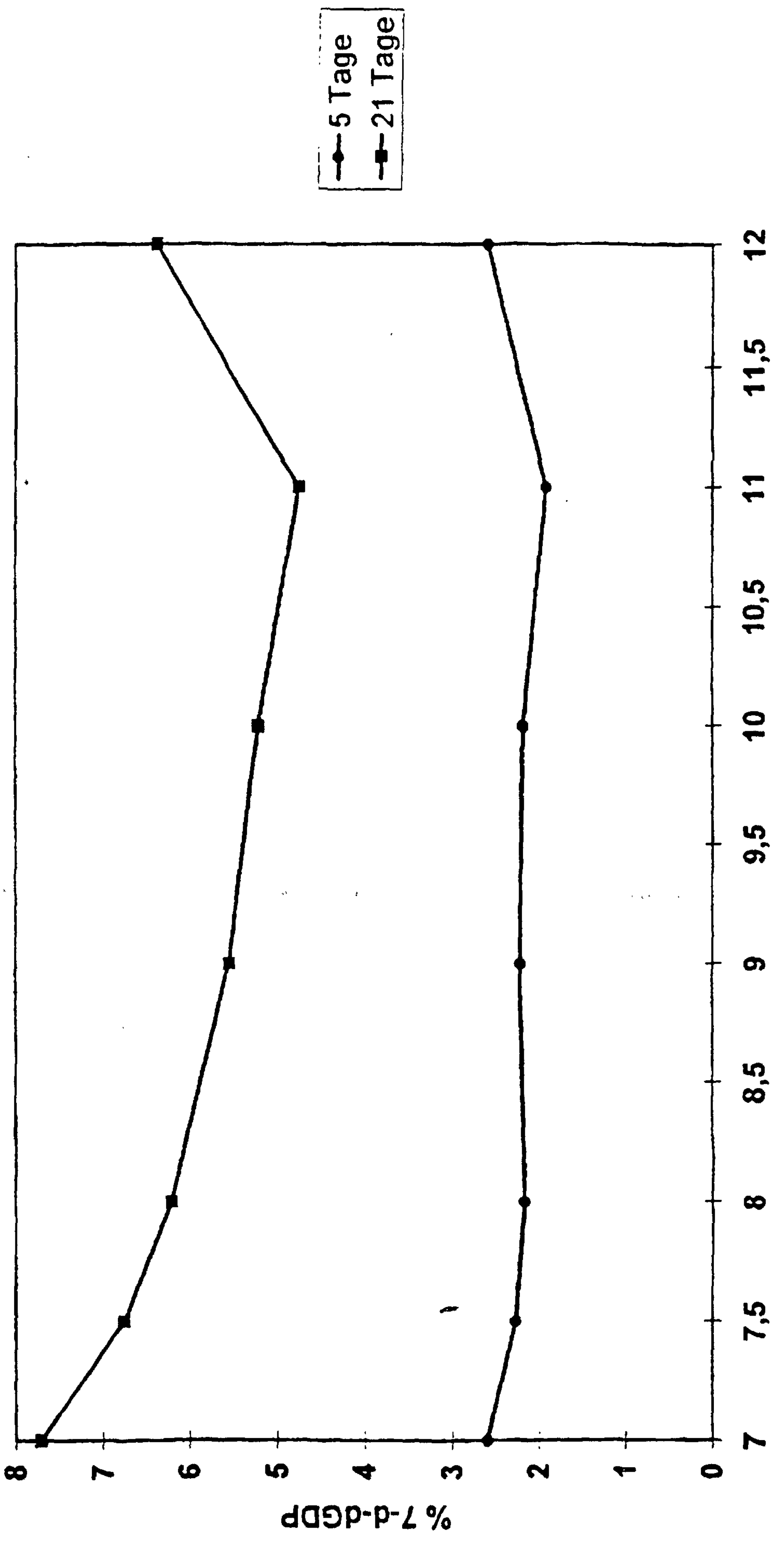

Abb. 13
Stabilität dATP bei Verdünnung

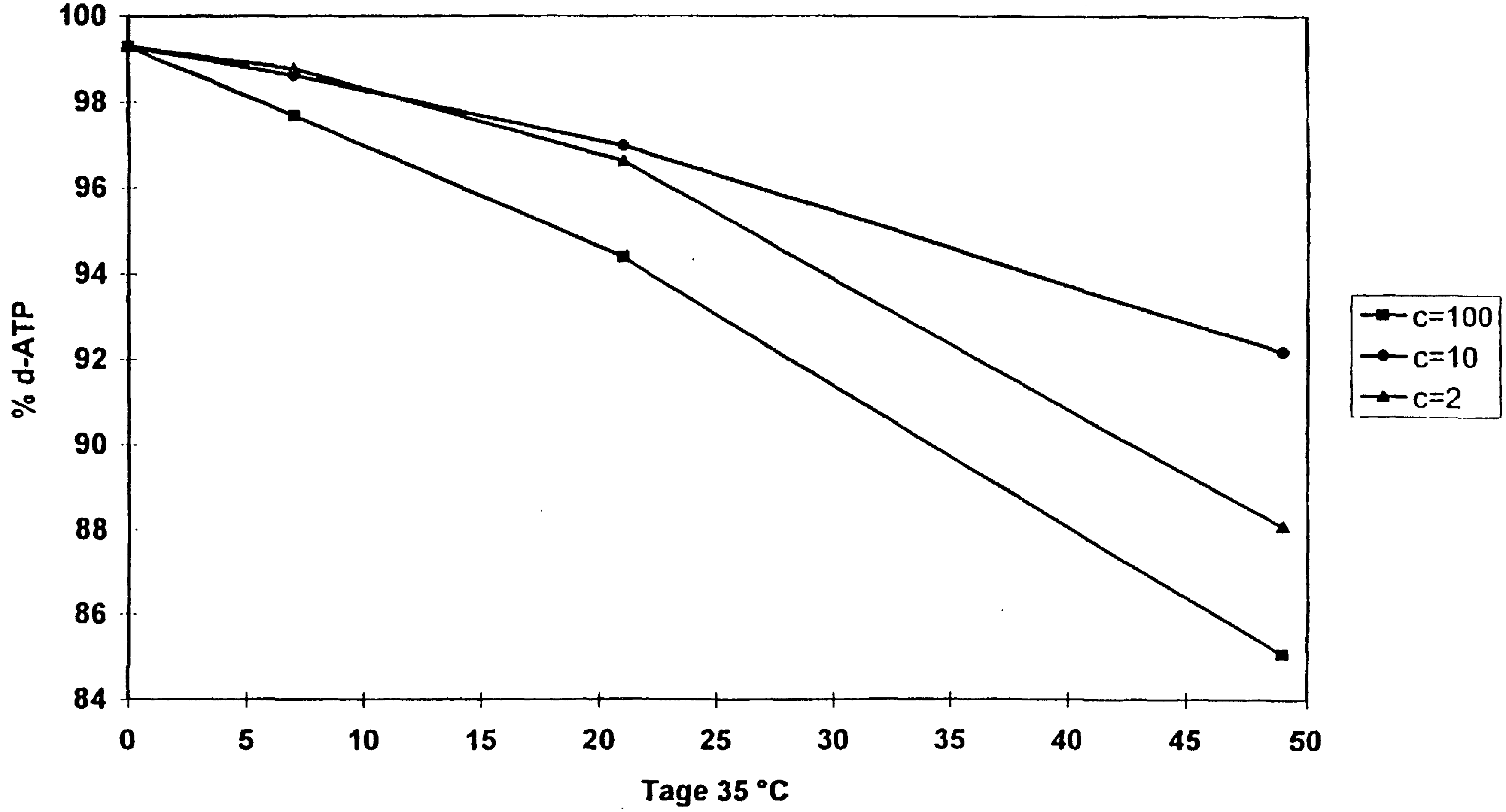

Abb. 14
Stabilität dATP

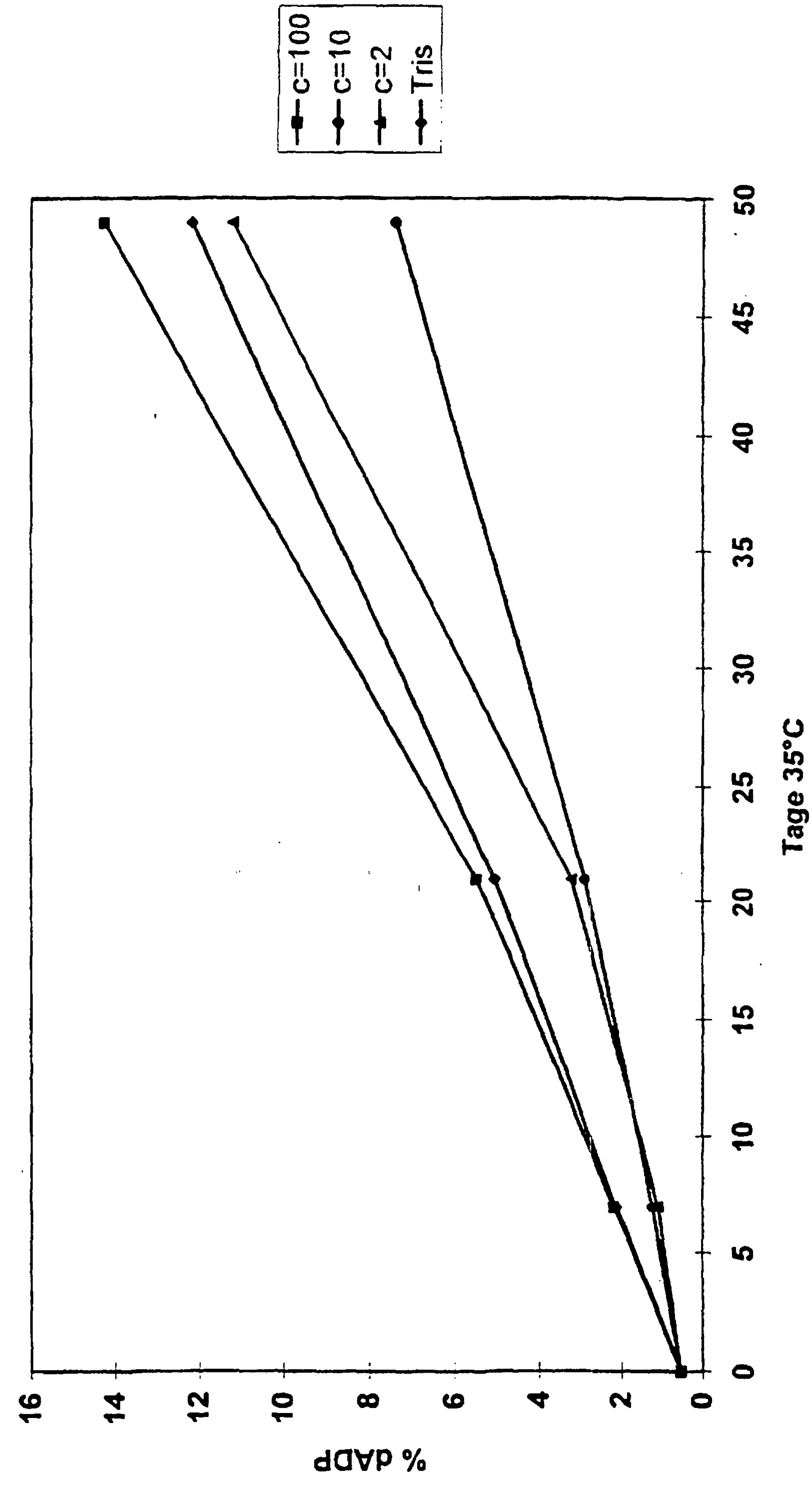

Abb. 15
Stabilität dCTP

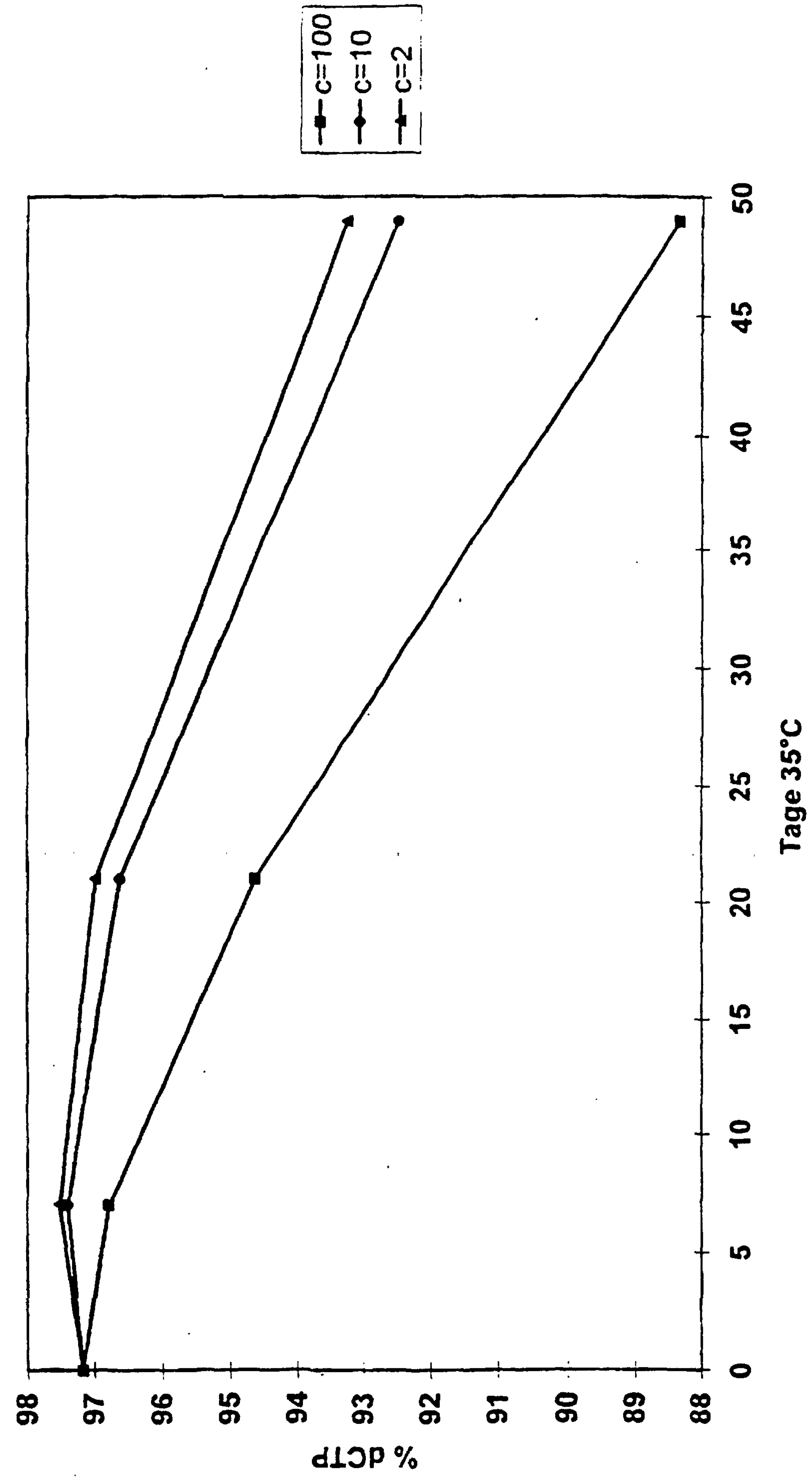

Abb. 16
Stabilität dCTP